(19) Europäisches Patentamt
European Patent Office
Office européen des brevets

(11) EP 0 526 267 B1

(12) **FASCICULE DE BREVET EUROPEEN**

(45) Date de publication et mention de la délivrance du brevet:
**13.08.1997 Bulletin 1997/33**

(51) Int Cl.[6]: **C07D 233/60**, C08F 226/06, C08F 8/44, A61K 7/06

(21) Numéro de dépôt: **92401746.0**

(22) Date de dépôt: **23.06.1992**

(54) **Composés polyfluoroalkylthiopoly(éthylimidazolium), procédé de préparation et leur utilisation comme agents biocides**

Polyfluoralkylthiopoly(ethylimidazolium)-Verbindungen, Verfahren zu ihrer Herstellung und ihre Verwendung als biozide Mittel

Polyfluoroalkylthiopoly(ethylimidazolium) compounds, process for their preparation, and their use as biocidal agents

(84) Etats contractants désignés:
**AT BE CH DE DK ES FR GB GR IT LI NL PT SE**

(30) Priorité: **24.06.1991 FR 9107734**

(43) Date de publication de la demande:
**03.02.1993 Bulletin 1993/05**

(73) Titulaire: **L'OREAL**
**75008 Paris (FR)**

(72) Inventeurs:
- **Vanlerberghe, Guy**
  **F-77410 Claye-Souilly (FR)**
- **Bollens, Eric**
  **F-94410 Saint-Maurice (FR)**
- **Mahieu, Claude**
  **F-75017 Paris (FR)**
- **Sebag, Henri**
  **F-75016 Paris (FR)**

(74) Mandataire: **Casalonga, Axel et al**
**BUREAU D.A. CASALONGA - JOSSE**
**Morassistrasse 8**
**80469 München (DE)**

(56) Documents cités:
**EP-A- 0 162 388**
**EP-A- 0 196 824**
**EP-A- 0 301 447**
**WO-A-90/04918**
**DE-A- 3 733 471**
**FR-A- 2 010 024**
**FR-A- 2 275 194**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 526 267 B1

Printed by Jouve, 75001 PARIS (FR)

## Description

La présente invention concerne de nouveaux composés du type polyfluoroalkylthiopoly(éthylimidazolium), leur procédé de préparation et leur utilisation comme agents biocides dans divers domaines techniques comme la cosmétique, la pharmacie humaine et vétérinaire, l'agriculture, les peintures et vernis, la papeterie.

On recherche en cosmétique des produits ayant des propriétés bactéricides et/ou fongicides et une bonne tolérance vis-à-vis de la peau et des cheveux, notamment dans les produits anti-pelliculaires, dans les produits de nettoyage de la peau.

En pharmacie, l'utilisation des produits bactéricides et/ou fongicides présente également un grand intérêt, notamment dans le traitement des maladies affectant la couche cornée de l'épiderme de l'homme ou de l'animal, tel que l'acné, dans le traitement des muqueuses ou dans le traitement des mycoses.

Les composés cationiques du type ammonium quaternaire sont couramment utilisés comme agents bactéricides en cosmétique ou pharmacie. Ces composés présentent cependant des problèmes de tolérance.

On connaît, dans l'état de la technique, le bromure de cétyltriméthylammonium plus connu sous le non de "CETAVLON".

FR-A-2 010 024 décrit des sels d'ammonium sulfurés et leur activité pour combattre les champignons, les algues ou les bactéries. Le document WO 90/04918 concerne des composés d'ammoniums quaternaires et leur utilisation dans des compositions thérapeutiques pour des traitements antiviraux. Les composés décrits ont une structure différente des composés selon la présente invention.

La demanderesse a découvert de nouveaux composés dérivés de l'imidazole, présentant une bonne activité biocide, ainsi qu'une toxicité amoindrie par rapport aux composés connus. Ils présentent de plus de bonnes propriétés cosmétiques vis-à-vis des cheveux, de la peau et des ongles.

Ces composés présentent par ailleurs des propriétés tensio-actives intéressantes.

La présente invention a pour objet de nouveaux composés cationiques du type imidazolium.

Un autre objet de l'invention consiste en un procédé de préparation de ces composés.

L'invention concerne également l'utilisation de ces composés comme agents biocides dans de nombreux domaines de l'industrie chimique, et plus particulièrement la cosmétique et la dermopharmacie.

D'autres objets apparaîtront à la lumière de la description et des exemples qui suivent.

Les composés conformes à la présente invention répondent à la formule suivante (I) :

$$CF_3 - (CF_2)_x - (CH_2)_y - \underset{(O)_w}{\underset{\|}{S}} - [C_5H_6N_2\, R^+\, X^-]_n - H \qquad (I)$$

dans laquelle :

- w est égal à 0, 1 ou 2;
- x est compris entre 2 et 10;
- y est compris entre 0 et 5;
- R désigne un radical méthyle, éthyle, hydroxyéthyle, benzyle;
- $X^{\ominus}$ désigne un anion minéral ou organique;
- n est un nombre entier ou décimal compris entre 1 et 15;

le groupement $[C_5H_6N_2\ R^+]$ représentant les structures suivantes prises en mélange ou séparément :

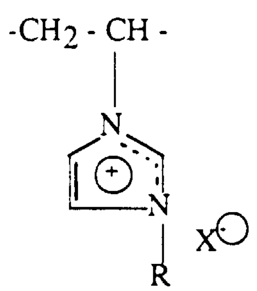

;

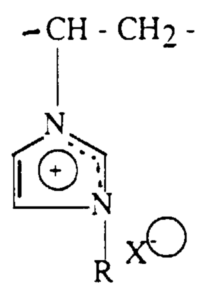

L'anion $X^{\ominus}$ désigne des halogénures, des alkylsulfates, des alkylsulfonates ou des arylsulfonates.

Il peut désigner notamment $Cl^-$, $Br^-$, $I^-$, $CH_3OSO_3^-$, $C_2H_5OSO_3^-$, $CH_3SO_3^-$, ou

$C_2H_5OSO_3^-$ , $CH_3SO_3^-$ ou 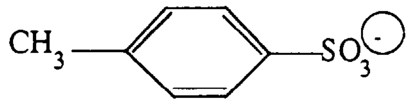

Les composés préférentiels selon la présente invention sont choisis parmi ceux de formule (I), dans laquelle w est égal à O.

Un autre objet de l'invention est constitué par un procédé de préparation des tensio-actifs cationiques de l'invention.

Les composés selon l'invention peuvent être préparés par addition radicalaire d'un mercaptan de formule :

$$CF_3 - (CF_2)_x - (CH_2)_y - SH$$

dans laquelle x et y ont la même désignation indiquée ci-dessus, sur une ou plusieurs molécule(s) de 1-vinylimidazole pour obtenir un composé de formule (II) suivante :

$$CF_3 - (CF_2)_x - (CH_2)_y - S - (C_5H_6N_2)_n - H \qquad (II)$$

dans laquelle le groupement ($C_5H_6N_2$) signifie les structures suivantes prises en mélange ou séparément :

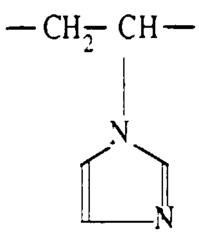 ; 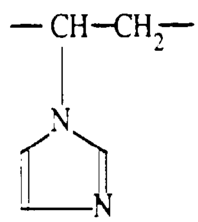

Le composé de formule (II) ainsi obtenu est ensuite quaternisé par alcoylation avec un composé de formule RX, dans laquelle R et X ont les significations indiquées ci-dessus.

Dans le cas où w = 1 ou 2, les produits ainsi obtenus sont oxydés à l'eau oxygénée selon un procédé connu, à une température comprise entre 20° et 50°C.

Le procédé de préparation des composés de l'invention peut être représenté par le schéma réactionnel suivant :

SCHEMA A

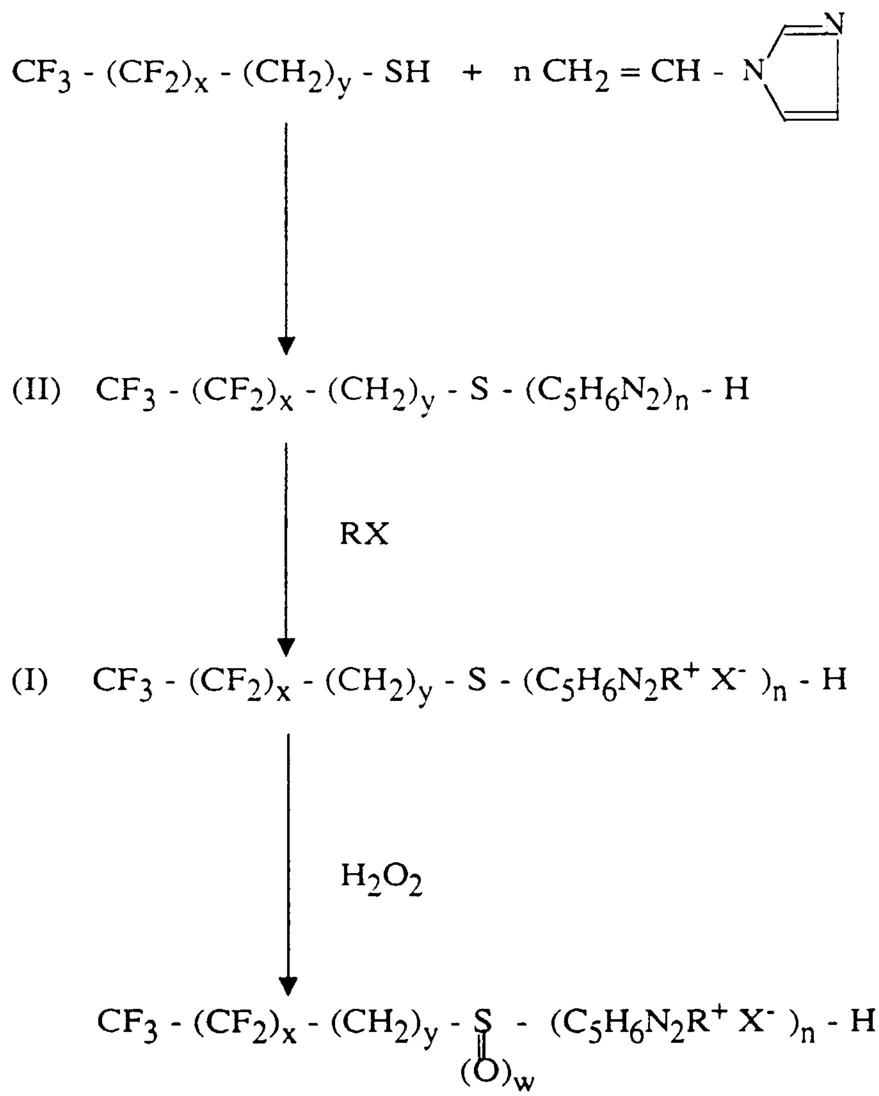


La réaction radicalaire a lieu en milieu solvant en présence d'un initiateur de radicaux libres.

Comme initiateurs de radicaux libres, on peut citer les hydroperoxydes tels que l'hydroperoxyde de tertiobutyle, les peroxydes tels que le peroxyde de dibenzoyle, les peresters tels que le peroxybenzoate de tertiobutyle ou les dérivés azoïques et en particulier l'azobisisobutyronitrile.

Les solvants utilisables doivent être inertes vis-à-vis des réactifs et peuvent être choisis parmi les alcools en $C_1$-$C_4$ tels que le méthanol, l'isopropanol, les alkyléthers, les éthers de glycol, les éthers cycliques tels que le tétrahydrofurane, les hydrocarbures aliphatiques ou aromatiques en $C_6$-$C_8$ comme le toluène.

Le mercaptan de départ est dissous dans le solvant en présence du 1-vinylimidazole, puis l'initiateur de radicaux est additionné; la réaction étant effectuée sous atmosphère inerte. Les composés de formule (II) ainsi obtenus sont ensuite alcoylés avec un alcoylant RX en présence d'un solvant inerte tel que ceux cités précédemment.

Les alcoylants RX utilisés conformément à l'invention sont choisis par exemple parmi les halogénures de méthyle ou d'éthyle, les sulfates de méthyle ou d'éthyle, le sulfonate de méthyle, le paratoluènesulfonate de méthyle ou le bromoéthanol.

Les composés de formule (II) sont nouveaux et constituent un autre objet de l'invention.

Les composés cationiques polyfluoroalkylthiopoly (éthylimidazolium) de formule (I) de l'invention, possèdent de bonnes propriétés biocides.

Une bonne activité biocide de ces composés a été observée selon les méthodes classiques sur les souches suivantes : Pseudomonas aeruginosa, Staphylococcus aureus, Candida albicans. Ces souches peuvent être considérées comme représentatives des principales bactéries et fongi responsables des affections cutanées d'origine bactérienne, mycobactérienne ou dues à l'implantation de levures pathogènes.

La faible toxicité des composés de l'invention a été observée par hémolyse des hématies d'échantillons de sang.

Les composés conformes à la présente invention peuvent être utilisés comme agents biocides ou comme agents de conservation dans le domaine de l'industrie chimique, notamment dans les produits cosmétiques et l'industrie pharmaceutique à usage humain ou vétérinaire, les produits agricoles, les produits de traitement des plantes, les peintures et les vernis, la papeterie.

Les composés de l'invention ont des propriétés tensio-actives intéressantes. De plus, ils ont la particularité de se fixer sur les matières kératiniques telles que la peau, les cheveux et les ongles.

Leur caractère amphiphile cationique leur confère en plus des propriétés de démêlage, de douceur, de brillance et de souplesse vis-à-vis des cheveux et des propriétés de douceur vis-à-vis de la peau.

D'autre part, la présence d'une chaîne perfluorée dans leur structure permet de conférer à la peau ou aux cheveux traités, une hydrophobie et une oléophobie.

Les composés selon l'invention permettent un séchage rapide des cheveux ou évitent un regraissage excessif de la peau et du cheveu.

Les composés de l'invention sont donc particulièrement intéressants pour les soins cosmétiques des matières kératiniques. Ils sont également particulièrement intéressants pour le traitement des affections cutanées d'origine bactérienne, mycobactérienne ou dues à l'implantation de levures pathogènes. Ils peuvent notamment être utilisés dans les compositions pharmaceutiques pouvant être appliquées par voie topique sur la peau ou sur les muqueuses, pour le traitement de l'acné ou des mycoses, ou dans des compositions cosmétiques, notamment des déodorants corporels ou des bains de bouche.

Un autre objet de l'invention consiste donc en des compositions pharmaceutiques ou cosmétiques pour le traitement ou le soin des matières kératiniques humaines, contenant dans un milieu physiologiquement acceptable, une quantité efficace de composés de formule (I) telle que définie précédemment.

Les composés de formule (I) sont présents dans des concentrations comprises de préférence entre 0,1 et 10% en poids par rapport au poids total de la composition et de préférence entre 0,2 et 5% en poids.

Ces compositions peuvent être des solutions aqueuses, alcooliques ou hydro-alcooliques, des émulsions se présentant sous forme de lait ou de crème, de mousse, de gel, de pâte, de stick ou encore sous forme de spray.

Ces compositions peuvent être pressurisées dans des dispositifs aérosols, en présence d'un agent propulseur, éventuellement en présence de générateurs de mousse ou d'agents émulsionnants.

Comme agents propulseurs, on peut citer les agents de type fréons, les alcanes en $C_3$-$C_5$, des solvants chlorés tels que le chlorure de méthylène ou les éthers comme le diméthyléther.

Les compositions peuvent également se présenter sous forme de dispersion vésiculaire à base de lipides ioniques (liposomes) ou non-ioniques.

Ces compositions contiennent de l'eau, un solvant organique physiologiquement acceptable ou un mélange d'eau et de ce solvant, le solvant étant choisi parmi les alcools inférieurs en $C_1$-$C_4$ tels que l'éthanol, l'isopropanol, le propanol ou les polyalcools tels que le propylèneglycol ou la glycérine; ces solvants étant présents dans des proportions comprises entre 0 et 50%.

Les compositions selon l'invention peuvent contenir également des huiles, des cires naturelles ou synthétiques, des alcools gras, des silicones, des produits tensio-actifs moussants, émulsionnants, dispersants, non-ioniques, cationiques, faiblement anioniques, amphotères ou zwittérioniques, des polymères d'origine naturelle comme les dérivés de cellulose, de guar, de chitosane, des peptides, des polymères de synthèse, des conditionneurs, des stabilisants de mousse, des épaississants, des nacrants, des stérols, des sels, des filtres solaires, des parfums, des colorants, des hydratants, des conservateurs autres que ceux de formule (I), notamment ceux de la famille des isothiazolones tels que le 2-méthylisothiazolone, 2-octyl isothiazolone, 5-chloro 2-méthylisothiazolone, benzo-isothiazolone ou celles décrites dans le brevet FR-2.492.376.

Une forme particulière d'application cosmétique selon l'invention, consiste en des compositions de lavage et/ou de traitement cosmétique des cheveux avec rinçage, à séchage rapide, contenant au moins un composé de formule (I) en présence d'agents détergents et moussants ou d'agents de traitements usuels compatibles avec les composés de l'invention.

Ces compositions peuvent se présenter sous forme de shampooing, d'après-shampooing ou de composition de rinçage des cheveux. Elles sont appliquées dans des quantités efficaces pour laver et/ou traiter les cheveux, puis suivies d'un rinçage à l'eau.

L'invention concerne également des compositions de traitement de la peau, renfermant au moins un composé de formule (I) en présence d'agents de traitements usuels compatibles avec les composés de l'invention, de manière à éviter un regraissage excessif de la peau après application.

Une autre forme de réalisation de l'invention consiste également en des compositions capillaires sous forme de shampooing ou de lotion pour l'élimination des pellicules.

Un autre objet de l'invention consiste en un procédé de traitement cosmétique des cheveux pour l'élimination des pellicules utilisant les compositions.

L'invention concerne également l'utilisation des composés de formule (I) pour la préparation d'un médicament pour traiter les affections cutanées d'origine bactérienne, mycobactérienne ou dues à des implantations de levures pathogènes.

Les exemples qui suivent servent à illustrer l'invention sans toutefois présenter un caractère limitatif.

EXEMPLES DE PREPARATION

EXEMPLE 1

Préparation d'un composé de formule (I) dans lequel :
x = 5 y=2 n=1 R=$CH_3$ X = $CH_3OSO_3^{\ominus}$ ; w = 0

ETAPE 1

Préparation d'un composé de formule (II) dans lequel :
x = 5 y=2 n=1

Dans un réacteur, on place 76 g de 2-F-hexyléthanethiol (0,2 mole) en solution dans 80 g de méthanol. Le mélange est agité sous atmosphère d'azote. 18,8 g de 1-vinylimidazole sont alors ajoutés en 5 minutes. Le chauffage est alors amorcé. Lorsque la température du mélange réactionnel atteint 55°C, une solution de 0,752 g d'azobisisobutyronitrile dans 40 g de méthanol est alors ajoutée goutte-à-goutte en 1 h 30 tout en poursuivant la montée en température. Lorsque l'addition est terminée, la température du mélange réactionnel est voisine de 66°C. Le méthanol est au reflux.

L'agitation, le chauffage et le courant d'azote sont maintenus pendant 14 heures.

Le mélange réactionnel est alors constitué des produits de mono-addition et de di-addition.

Le produit de mono-addition est isolé par filtration sur silice 60 H (éluant $CH_2Cl_2/CH_3OH$: 95/5) avec un rendement de 80% (m=76 g).

Indice de basicité : 2,07 meq/g (théorique : 2,10 meq/g)
Indice de thioéther : 2,05 meq/g (théorique : 2,10 meq/g)

| ANALYSE ELEMENTAIRE | | |
|---|---|---|
| | THEORIQUE | TROUVE |
| C | 32,92 | 32,69 |
| H | 2,34 | 2,45 |
| N | 5,91 | 5,84 |
| S | 6,76 | 7,10 |
| F | 52,08 | 51,84 |

ETAPE 2

Quaternisation du composé de l'étape 1.

Dans un réacteur, on dissout 23,7 g (0,05 mole) du composé de l'étape 1 dans 25 ml de méthanol.

A 25°C, on additionne goutte-à-goutte en 1 heure, 6,19 g de sulfate de diméthyle en évitant que la température du mélange réactionnel ne dépasse pas 35°C.

Le mélange est agité 14 heures à température ambiante. Le solvant est ensuite évaporé sous pression réduite. On obtient 30,70 g d'une pâte brun clair.

| ANALYSE ELEMENTAIRE | | |
|---|---|---|
| | THEORIQUE | TROUVE |
| C | 30,01 | 29,74 |

(suite)

| ANALYSE ELEMENTAIRE | | |
|---|---|---|
| | THEORIQUE | TROUVE |
| H | 2,85 | 3,05 |
| N | 4,67 | 4,41 |
| S | 10,68 | 10,34 |
| F | 41,14 | 40,83 |

EXEMPLE 2

Composé de formule (I) dans lequel :
x = 5 y = 2 = 2 = $CH_3$ X = $CH_3OSO_3^{\ominus}$ ; w =0

ETAPE 1

Composé de formule (II) dans lequel :
x = 5 y=2 n=2

Ce composé est préparé selon le mode opératoire de l'exemple 1, étape 1.

Le produit de dicondensation est isolé du mélange réactionnel par filtration sur silice 60 H (éluant $CH_2Cl_2/CH_3OH$ : 95/5).

On obtient parallèlement aux 76 g de 2-(2'-F-hexyléthylthio)éthylimidazole (exemple 1), 5 g de produit de dicondensation, le 2-[2'-(2"-F hexyléthylthio)éthylimidazole]éthylimidazole.

Le produit se présente sous la forme d'une pâte brun clair.

Indice de basicité : 3,45 meq/g (théorique : 3,52 meq/g)
Indice de thioéther : 1,70 meq/g (théorique: 1,76 meq/g)

| ANALYSE ELEMENTAIRE | | |
|---|---|---|
| | THEORIQUE | TROUVE |
| C | 38,03 | 37,16 |
| H | 3,01 | 3,25 |
| N | 9,86 | 9,58 |
| S | 5,64 | 5,56 |
| F | 43,45 | 41,30 |

ETAPE 2

Quaternisation du composé de l'étape 1

Le mode opératoire est analogue à celui de l'exemple 1, étape 2, en utilisant :

- 3,15 g du composé de l'étape 1 solubilisés dans 5 g de méthanol;
- 1,37 g de sulfate de diméthyle.

On obtient 4,5 g de produit quaternisé qui se présente sous la forme d'une pâte beige.

| ANALYSE ELEMENTAIRE | | |
|---|---|---|
| | THEORIQUE | TROUVE |
| C | 32,20 | 31,48 |
| H | 3,56 | 3,78 |
| N | 6,83 | 6,50 |
| S | 11,72 | 10,90 |

(suite)

| ANALYSE ELEMENTAIRE | | |
|---|---|---|
| | THEORIQUE | TROUVE |
| F | 30,10 | 31,86 |

EXEMPLE 3

Préparation d'un composé de formule (I) dans lequel :
x = 5 y = 2 $\bar{n}$ = 3 R = $CH_3$ X = $CH_3OSO_3^{\ominus}$ ; w = 0

ETAPE 1

Préparation d'un composé de formule (II) dans lequel :
x=5 y=2 $\bar{n}$=3
Le mode opératoire est analogue à celui de l'exemple 1, étape 1, en utilisant :

- 38 g de 2-F-hexyléthanethiol (0,1 mole) dissous dans 40 g de méthanol;
- 29,61 g (0,3 mole) de 1-vinylimidazole;
- 1,12 g d'azobisisobutyronitrile dans 40 g de méthanol.

Après réaction, le solvant est évaporé sous pression réduite et l'on obtient 67,5 g d'une pâte beige clair.

Indice de basicité : 4,47 meq/g (théorique : 4,53 meq/g)
Indice de thioéther : 1,60 meq/g (théorique : 1,51 meq/g)

ETAPE 2

Quaternisation du composé de l'étape 1.

Le mode opératoire est analogue à celui de l'exemple 1, étape 2, en utilisant :

- 20 g du composé de l'étape 1 dissous dans 50 ml de méthanol;
- 11,26 g de sulfate de diméthyle.

| ANALYSE ELEMENTAIRE | | |
|---|---|---|
| | THEORIQUE | TROUVE |
| C | 33,61 | 33,34 |
| H | 4,02 | 4,49 |
| N | 8,22 | 8,36 |
| S | 12,39 | 12,15 |
| F | 23,00 | 21,55 |

EXEMPLE 4

Préparation d'un composé de formule (II) dans lequel :
x =7 y = 2 = 1 = $CH_3$ X = $CH_3OSO_3^{\ominus}$ ; w =0

ETAPE 1

Préparation d'un composé de formule (II) dans lequel :
x=7 y=2 n= 1
Le mode opératoire est analogue à celui de l'exemple 1, étape 1, en utilisant :

- 96 g de 2-F-octyléthanethiol (0,2 mole) dissous dans 80 g de méthanol;

- 18,8 g de 1-vinylimidazole (0,2 mole);
- 0,752 g d'azobisisobutyronitrile dans 40 g de méthanol.

Le mélange réactionnel est constitué des produits de monoaddition et de diaddition.

Le produit de monoaddition, le 2-(2'-F-octyléthylthio)éthylimidazole est isolé par filtration sur silice Merck 60 H dans les mêmes conditions que pour l'exemple 1, étape 1.

Rendement = 85% (m = 98 g)

| | |
|---|---|
| Indice de basicité : | 1,69 meq/g (théorique : 1,74 meq/g) |
| Indice de thioéther : | 1,75 meq/g (théorique : 1,74 meq/g) |

| ANALYSE ELEMENTAIRE | | |
|---|---|---|
| | THEORIQUE | TROUVE |
| C | 31,36 | 31,09 |
| H | 1,92 | 1,90 |
| N | 4,88 | 4,89 |
| S | 5,57 | 5,45 |
| F | 56,27 | 55,86 |

ETAPE 2

Quaternisation du composé de l'étape 1.

Le mode opératoire est analogue à celui de l'exemple 1, étape 2, en utilisant :

- 43,05 g (0,075 mole) de composé de l'étape 1 dissous dans 40 ml de méthanol;
- 9,45 g de sulfate de diméthyle.

On obtient 52,50 g d'une pâte marron clair.

| ANALYSE ELEMENTAIRE | | |
|---|---|---|
| | THEORIQUE | TROUVE |
| C | 29,14 | 28,89 |
| H | 2,43 | 2,59 |
| N | 4,00 | 3,74 |
| S | 9,14 | 9,11 |
| F | 46,14 | 45,84 |

EXEMPLE 5

Préparation d'un composé de formule (I) dans lequel :

x = 7 y = 2 $\bar{n}$ = 1,5 R = $CH_3$ X = $CH_3OSO_3^{\ominus}$ ; w =0

ETAPE 1

Préparation d'un composé de formule (II) dans lequel :

x=7 y=2 $\bar{n}$ = 1,5

Le mode opératoire est analogue à celui de l'exemple 1, étape 1, en utilisant :

- 96 g de 2-F-octyléthanethiol (0,2 mole) dissous dans 80 g de méthanol;
- 28,2 g (0,3 mole) de 1-vinylimidazole;
- 1,15 g d'azobisisobutyronitrile dans 40 g de méthanol.

On obtient 124 g d'un produit pâteux de couleur beige.

Indice de basicité : 2,41 meq/g (théorique : 2,41 meq/g)
Indice de thioéther : 1,60 meq/g (théorique: 1,61 meq/g)

| ANALYSE ELEMENTAIRE | | |
|---|---|---|
| | THEORIQUE | TROUVE |
| C | 33,83 | 33,69 |
| H | 2,27 | 2,40 |
| N | 6,76 | 6,81 |
| S | 5,16 | 4,95 |
| F | 41,98 | 42,08 |

ETAPE 2

Quaternisation du composé de étape 1.

Le mode opératoire est analogue à celui de l'exemple 1, étape 2, en utilisant :

- 30 g de composé de l'étape 1 dissous dans 37 ml de méthanol;
- 9,11 g de sulfate de diméthyle.

On obtient 39 g de produit pâteux de couleur beige clair.

Indice de basicité : 0,1 meq/g.

| ANALYSE ELEMENTAIRE | | |
|---|---|---|
| | THEORIQUE | TROUVE |
| C | 30,38 | 30,31 |
| H | 2,86 | 2,86 |
| N | 5,18 | 5,06 |
| S | 9,89 | 10,09 |
| F | 39,95 | 39,55 |

EXEMPLE 6

Préparation d'un composé de formule (II) dans lequel :
$x = y = 2$ $\bar{n} = 3 = CH_3$ $X = CH_3OSO_3^{\ominus}$ ; $w = 0$

ETAPE 1

Préparation d'un composé de formule (II) dans lequel :
$x = 7$ $y = 2$ $\bar{n} = 3$
Le mode opératoire est analogue à celui de l'exemple 1, étape 1, en utilisant :

- 96 g (0,2 mole) de 2-F-octyléthanethiol dissous dans 76 g d'isopropanol;
- 56,4 g (0,6 mole) de 1-vinylimidazole;
- 2,24 g d'azobisisobutyronitrile dans 150 g d'isopropanol.

Indice de basicité : 3,83 meq/g (théorique : 3,93 meq/g)
Indice de thioéther : 1,34 meq/g (théorique: 1,31 meq/g)

| ANALYSE ELEMENTAIRE | | |
|---|---|---|
| | THEORIQUE | TROUVE |
| C | 39,59 | 39,34 |
| H | 2,72 | 3,18 |
| N | 10,31 | 10,74 |
| S | 3,92 | 4,30 |
| F | 39,46 | 40,87 |

ETAPE 2

Quaternisation du composé de l'étape 1.

Le mode opératoire est analogue à celui de l'exemple 1, étape 2, en utilisant :

- 40 g de composé de l'étape 1 dissous dans 30 ml de méthanol;
- 19,3 g de sulfate de diméthyle.

On obtient 59,3 g de produit beige clair.

| ANALYSE ELEMENTAIRE | | |
|---|---|---|
| | THEORIQUE | TROUVE |
| C | 32,63 | 32,42 |
| H | 3,62 | 3,76 |
| N | 7,37 | 7,21 |
| S | 11,24 | 10,99 |
| F | 28,31 | 28,58 |

EXEMPLE 7

Préparation d'un composé de formule (I) dans lequel :
x = 7 y = 2 $\bar{n}$ = 5 X = $CH_3OSO_3^{\ominus}$ R = $CH_3$ ; w = 0

ETAPE 1

Prépararation d'un composé de formule (II) dans lequel :
x=7 y=2 $\bar{n}$ = 5
Le mode opératoire est analogue à celui de l'exemple 1, étape 1, en utilisant :

- 48 g de 2-F-octyléthanethiol (0,1 mole) dissous dans 85 ml de méthanol;
- 47 g (0,5 mole) de 1-vinylimidazole;
- 1,88 g d'azobisisobutyronitrile dans 60 ml de méthanol.

On obtient 103,2 g d'un produit de couleur beige.

Indice de basicité : 4,62 meq/g (théorique : 5,26 meq/g)
Indice de thioéther : 1,11 meq/g (théorique : 1,05 meq/g)

| ANALYSE ELEMENTAIRE | | |
|---|---|---|
| | THEORIQUE | TROUVE |
| C | 44,21 | 44,56 |
| H | 3,71 | 4,74 |

(suite)

| ANALYSE ELEMENTAIRE | | |
|---|---|---|
| | THEORIQUE | TROUVE |
| N | 14,73 | 14,96 |
| S | 3,37 | 4,04 |
| F | 33,97 | 32,01 |

ETAPE 2

Quaternisation du composé de l'étape 1.

Le mode opératoire est analogue à celui de l'exemple 1, étape 2, en utilisant :

- 30 g de composé de l'étape 1 dissous dans 100 ml de méthanol;
- 17,47 g de sulfate de diméthyle.

On obtient 47,5 g de produit beige clair.

EXEMPLE 8

Préparation d'un composé de formule (I) dans lequel :
x = 7 y = 2 n = 2 X = $CH_3OSO_3^{\ominus}$ R = $CH_3$ ; w = 0

ETAPE 1

Préparation d'un composé de formule (II) dans lequel :
x=7 y=2 n = 2

Le mode opératoire est analogue à celui de l'exemple 4, étape 1.

Le produit de dicondensation est isolé du mélange réactionnel par filtration sur silice 60 H (éluant $CH_2Cl_2/CH_3OH$ : 95/5).

On obtient parallèlement aux 98 g de 2-(2'-F-octyléthylthio) éthylimidazole (exemple 4), 7 g de produit de dicondensation, le 2- [2'-F-octyléthylthio)éthylimidazole]éthylimidazole.

Le produit se présente sous la forme d'une pâte brun foncé.

Indice de basicité : 2,79 meq/g (théorique : 2,99 meq/g)
Indice de thioéther : 1,37 meq/g (théorique: 1,49 meq/g)

| ANALYSE ELEMENTAIRE | | |
|---|---|---|
| | THEORIQUE | TROUVE |
| C | 35,95 | 35,14 |
| H | 2,50 | 2,70 |
| N | 8,38 | 7,89 |
| S | 4,79 | 4,72 |
| F | 48,35 | 47,54 |

ETAPE 2

Quaternisation du composé issu de l'étape 1.

Le mode opératoire est analogue à celui de l'exemple 1, étape 2, en utilisant :

- 6,5 g du composé de l'étape 1 solubilisés dans 10 g de méthanol;
- 2,22 g de sulfate de diméthyle.

On obtient 8,7 g de produit quaternisé qui se présente sous la forme d'une pâte beige foncée.

| ANALYSE ELEMENTAIRE | | |
|---|---|---|
| | THEORIQUE | TROUVE |
| C | 31,30 | 30,59 |
| H | 3,15 | 3,43 |
| N | 6,09 | 5,78 |
| S | 10,43 | 10,04 |
| F | 35,15 | 33,91 |

EXEMPLE 9

Préparation d'un composé de formule (I) dans lequel :
x = 7 =2 $\bar{n}$ = 10 X = $CH_3OSO_3^{\ominus}$ R = $CH_3$ ; w = 0

ETAPE 1

Préparation d'un composé de formule (II) dans lequel :
x=7 y=2 $\bar{n}$ = 10

Le mode opératoire est analogue à celui de l'exemple 1, étape 1, en utilisant :

- 20 g de 2-F-octyléthanethiol (0,0417 mole) dissous dans 100 ml de méthanol;
- 39,17 g de 1-vinylimidazole;
- 1,57 g d'azobisisobutyronitrile.

Après réaction, le solvant est évaporé sous pression réduite et on obtient 59 g d'une pâte beige clair.

Indice de basicité : 6,08 meq/g (théorique : 7,04 meq/g)

| ANALYSE ELEMENTAIRE | | |
|---|---|---|
| | THEORIQUE | TROUVE |
| C | 50,70 | 48,22 |
| H | 4,61 | 5,37 |
| N | 19,71 | 18,55 |
| S | 2,26 | 2,02 |
| F | 22,72 | 22,88 |

ETAPE 2

Quaternisation du composé de l'étape 1.

Le mode opératoire est analogue à celui de l'exemple 1, étape 2, en utilisant :

- 20 g du composé de l'étape 1 dissous dans 60 ml de méthanol;
- 15,32 g de sulfate de diméthyle.

On obtient 34,5 g de produit.

| ANALYSE ELEMENTAIRE | | |
|---|---|---|
| | THEORIQUE | TROUVE |
| C | 35,82 | 34,25 |
| H | 4,70 | 5,29 |

(suite)

| ANALYSE ELEMENTAIRE | | |
|---|---|---|
| | THEORIQUE | TROUVE |
| N | 10,44 | 10,05 |
| S | 13,15 | 11,80 |
| F | 12,04 | 11,99 |

EXEMPLE 10

Préparation d'un composé de formule (I) dans lequel :
$x = 5$, $y = 2$, $n = 1$, R = benzyle, $X = Cl^-$ ; $w = 0$

Dans un réacteur de 500 ml, on solubilise 100 g (0,211 mole) du composé obtenu à l'étape 1 de l'exemple 1 dans 270 ml de diméthyl acétamide. On additionne goutte à goutte 26,7 g de chlorure de benzyle en 5 minutes.

Le mélange est chauffé 20 heures à 70°C.

Après retour à température ambiante, la solution est additionnée goutte à goutte en 10 minutes à 800 ml de tétrahydrofuranne. On obtient un précipité jaune pâle qui est filtré et lavé par deux fois 300 ml de tétrahydrofuranne chauffé à 60°C.

Après séchage sous vide à l'étuve, on obtient 88 g (70%) d'un solide blanc dont le spectre RMN $^{13}C$ est conforme à la structure attendue.

Le point de fusion du chlorure de N-(3-thio-5-F-hexyl)-pentyl benzylimidazolium est de 124°C.

| ANALYSE ELEMENTAIRE | | |
|---|---|---|
| | THEORIQUE | TROUVE |
| C | 39,98 | 39,87 |
| H | 3,02 | 3,07 |
| N | 4,66 | 4,62 |
| S | 5,34 | 5,32 |
| Cl | 5,90 | 5,88 |
| F | 41,10 | 41,05 |

EXEMPLE 11

Préparation d'un composé de formule (I) dans lequel :
$x = 5$, $y = 2$, $n = 1$, $R = C_2H_5$, $X = Br^-$ ; $w = 0$

Dans un réacteur de 500 ml, on solubilise 40 g (0,84 mole) du composé obtenu à l'étape 1 de l'exemple 1 dans 160 ml de tétrahydrofuranne. Lorsque le réactif est parfaitement solubilisé, on additionne 18,4 g (0,168 mole) de bromure d'éthyle en 5 minutes. Ce mélange est ensuite chauffé 36 heures au reflux du solvant.

Le chauffage est alors arrêté. Lorsque le mélange est revenu à 25°C, on observe la présence de deux phases.

La phase supérieure est éliminée par décantation. Après trois lavages successifs avec 100 ml de tétrahydrofuranne suivis de l'élimination du solvant par décantation, puis du séchage sous vide à l'étuve du résidu, on obtient 32 g d'une pâte orangée soluble dans l'eau (70%) dont le spectre RMN $^{13}C$ est conforme à la structure attendue.

| ANALYSE ELEMENTAIRE | | |
|---|---|---|
| | THEORIQUE | TROUVE |
| C | 30,89 | 30,05 |
| H | 2,77 | 2,81 |
| N | 4,80 | 4,73 |
| S | 5,50 | 5,68 |
| Br | 13,70 | 13,37 |
| F | 42,35 | 40,65 |

EXEMPLE 12

Préparation d'un composé de formule (I) dans lequel :
$x = 5$, $y = 2$ $\bar{n} = 3$, R = benzyle, X = $Cl^-$ ; w = 0

On solubilise 40 g du composé statistique obtenu à l'étape 1 de l'exemple 3 dans 200 ml de diméthylacétamide à 80°C.

Lorsque tout le produit est passé en solution, on ajoute à 80°C 61,3 g de chlorure de benzyle en 15 minutes. Le mélange est alors chauffé 24 heures à 80°C puis ramené à 25°C, température à laquelle le produit est précipité sous agitation par addition goutte à goutte dans 900 ml de tétrahydrofuranne.

Le produit est alors filtré, puis lavé au tétrahydrofuranne (2 fois 300 ml) puis séché.

Le précipité, hygroscopique, est repris par 600 ml d'eau. Après l'avoir glacée dans un bain d'éthanol + carboglace, la solution est alors lyophilisée.

Après cette opération, on obtient 44 g d'une poudre beige sèche.

| ANALYSE ELEMENTAIRE | | |
|---|---|---|
| | THEORIQUE | TROUVE |
| C | 50,70 | 49,40 |
| H | 4,26 | 4,98 |
| N | 8,06 | 8,35 |
| S | 3,08 | 1,90 |
| Cl | 10,21 | 10,21 |
| F | 23,70 | 21,87 |

EXEMPLE 13

Préparation d'un composé de formule (I) dans lequel :
x = 5, y = 2, n = 1, R = $CH_3$, X=$Cl^-$ ; w = 0

Dans un réacteur de 1500 ml, on introduit 200 g du composé obtenu à l'étape 1 de l'exemple 1 en solution dans 500 ml de diméthyl acétamide. Le réacteur est alors fermé hermétiquement puis, sous agitation, la solution est chauffée à 75°C. Lorsque cette température est atteinte, du chlorure de méthyle à l'état gazeux est introduit dans le réacteur jusqu'à ce que la pression à l'intérieur du système atteigne 1500 mbar.

Lorsque cette pression est obtenue, l'arrivée de chlorure de méthyle est stopée jusqu'à consommation complète du gaz introduit (retour à la pression atmosphérique à l'intérieur du réacteur). Lorsque le système ne consomme plus de chlorure de méthyle, l'installation est purgée à l'azote. Après retour à 25°C, le produit est précipité dans 1,5 litre d'acétate d'éthyle sous vive agitation.

Après élimination du solvant par décantation, le résidu obtenu est lavé trois fois par 200 ml d'acétate d'éthyle. Après séchage à l'étuve, on obtient 153 g (70%) d'une pâte beige dont le spectre RMN $^{13}C$ est conforme à la structure attendue.

| ANALYSE ELEMENTAIRE | | |
|---|---|---|
| | THEORIQUE | TROUVE |
| C | 32,04 | 30,59 |
| H | 2,69 | 3,25 |
| N | 5,33 | 5,33 |
| S | 6,11 | 5,91 |
| Cl | 16,76 | 5,88 |
| F | 47,06 | 46,96 |

EXEMPLE 14

Préparation d'un composé de formule (I) dans lequel :
x = 5, y = 2, n = 1, R = hydroxyéthyle, X = $Br^-$ ; w = 0

Dans un réacteur de 500 ml, on solubilise 47,4 g du composé obtenu à l'étape 1 de l'exemple 1 dans 135 ml de diméthylacétamide. On additionne goutte à goutte 50 g (0,4 ml) de bromoéthanol en 10 mimutes.

Le mélange est chauffé 48 heures à 70°C.

Après retour à la température de 25°C, la solution est précipitée dans 200 ml d'acétate d'éthyle. On obtient un précipité beige pâteux. La phase surnageante est éliminée par décantation et le résidu lavé avec deux fois 200 ml d'acétate d'éthyle.

Le produit est reprécipité dans 100 ml d'acétate d'éthyle à 70°C. Après retour à 25°C, le solvant est éliminé par décantation.

Après séchage à l'étuve, on obtient 35 g d'un produit pâteux beige (50%). Le produit est purifié par chromatographie en phase liquide sur silice Merck 60 H (éluant $CH_2Cl_2/CH_3$ OH : 90/10).

Le spectre RMN $^{13}C$ est conforme à la structure attendue.

EXEMPLE A

**EMULSION HUILE-DANS-EAU**

- Polyisobutène hydrogéné 6,5 g
- Palmitate d'octyle 5,0 g
- Cyclométhicone 5,0 g
- Alcool cétylique 4,0 g
- Stéarate de glycérol 3,0 g
- Glycérine 3,0 g
- Stéarate de polyéthylèneglycol oxyéthyléné à 40 moles d'oxyde d'éthylène, vendu sous la dénomination "MYRJ 52" par la Société ICI 2,0 g
- Myristate de myristyle 2,0 g
- Tristéarate de sorbitan 0,9 g
- Sel dipotassique de l'acide éthylène diamine tétracétique 0,05 g
- Composé de l'exemple 5 5,0 g
- Conservateur qs
- Eau qsp 100,0 g

EXEMPLE B

**EMULSION HUILE-DANS-EAU**

- Polyisobutène hydrogéné 6,5 g
- Palmitate d'octyle 5,0 g
- Cyclométhicone 5,0 g
- Alcool cétylique 4,0 g
- Stéarate de glycérol 3,0 g
- Glycérine 3,0 g
- Stéarate de polyéthylèneglycol oxyéthyléné à 40 moles d'oxyde d'éthylène, vendu sous la dénomination "MYRJ 52" par la Société ICI 2.0 g
- Myristate de myristyle 2,0 g
- Tristéarate de sorbitan 0,9 g
- Sel dipotassique de l'acide éthylène diamine tétracétique 0,05 g
- Composé de l'exemple 7 2,5 g
- Conservateur qs
- Eau qsp 100,0 g

EXEMPLE C

**EMULSION EAU-DANS-HUILE**

- Vaseline 14,2 g
- Huile de vaseline 12,2 g
- Lanoline hydrogénée 6,3 g
- Palmitate d'isopropyle 4,5 g
- Mélange d'octanoate de cétéaryle et de myristate d'isopropyle, commercialisé sous la dénomination "PCL Liquide huile 2/066210" par la Société DRAGOCO 2,85 g

- Lanolate de magnésium 2,7 g
- Palmitate d'octoxyglycéryle 1,9 g
- Huile de palme 0,5 g
- 5-chloro-2-(2,4-dichlorophénoxy)phénol, commercialisé sous la dénomination "TRICLOSAN" par la Société CIBA-GEIGY 0.5 g
- Alcool de lanoline vendu sous la dénomination "HARTOLAN" par la Société CRODA 0,43 g
- Composé de l'exemple 5 5,0 g
- Parfum qs
- Conservateur qs
- Eau qsp 100,0 g

EXEMPLE D

**EMULSION EAU-DANS-HUILE**

- Vaseline 14,2 g
- Huile de vaseline 12,2 g
- Lanoline hydrogénée 6,3 g
- Palmitate d'isopropyle 4,5 g
- Mélange d'octanoate de cétéaryle et de myristate d'isopropyle, commercialisé sous la dénomination "PCL Liquide huile 2/066210 par la Société DRAGOCO 2,85 g
- Lanolate de magnésium 2,7 g
- Palmitate d'octoxyglycéryle 1,9 g
- Huile de palme 0,5 g
- 5-chloro-2-(2,4-dichlorophénoxy)phénol, commercialisé sous la dénomination "TRICLOSAN" par la Société CIBA-GEIGY 0,5 g
- Alcool de lanoline vendu sous la dénomination "HARTOLAN" par la Société CRODA 0,43 g
- Composé de l'exemple 7 2,5 g
- Parfum qs
- Conservateur qs
- Eau qsp 100,0 g

EXEMPLE E

**DEODORANT VAPORISATEUR**

- Composé de l'exemple 7 1,0 g
- Eau 50,0 g
- Parfum 1,0 g
- Alcool éthylique à 95° qsp 100,0 g

EXEMPLE F

**DEODORANT STICK AQUEUX**

- Composé de l'exemple 3 0,23 g
- Stéarate de sodium 7.5 g
- Propylèneglycol 65,0 g
- Parfum 1,0 g
- Eau qsp 100.0 g

EXEMPLE G

**SHAMPOOING**

- Alkylpolyglycoside vendu sous la dénomination "APG 300 CS" en solution à 50% de matière active (MA) 15.0 g MA
- Composé de l'exemple 9 5,0 g

- Eau qsp 100,0 g
- pH spontané = 5

EXEMPLE H

SHAMPOOING

- Laurylsarcosinate de sodium vendu sous la dénomination "ORAMIX L30" par la Société SEPPIC 15,0 g
- Cocobétaïne vendu sous la dénomination "DEHYTON AB30" par la Société HENKEL 3,75 g
- Composé de l'exemple 3 0,2 g
- Eau qsp 100,0 g
- pH spontané = 7,2

EXEMPLE I

**SHAMPOOING**

- Composé de l'exemple 4 2 g
- Alkylpolyglycoside vendu sous la dénomination "APG 300" par la Société HENKEL 15,0 g MA
- Sel disodique de l'acide éthylènediamine tétracétique 0,5 g MA
- Conservateurs 0,3 g
- Parfum 0,5 g
- pH = 6 qs
- Eau purifiée qsp 100,0 g

Le shampooing permet un séchage rapide des cheveux humides.

EXEMPLE J

**APRES-SHAMPOOING RINCE**

- Composé de l'exemple 5 1,0 g
- Mélagne d'alcool cétyl stéarylique et d'alcool cétyl stéarylique oxyéthyléné à 33 moles d'oxyde d'éthylène, vendu sous la dénomination "SINNOWAX AO" par la Société HENKEL 3,0 g
- Ammonium diméthyl dialkyl chlorure 2,3 g
- Colorant 0,017 g
- Conservateur 0,04 g
- pH = 3,5 qs
- Eau purifiée qsp 100,0 g

Cet après-shampooing rincé permet un séchage rapide des cheveux humides.

EXEMPLE K

**EMULSION HUILE-DANS-EAU**

- Polyisobutène hydrogéné 6,5 g
- Palmitate d'octyle 5,0 g
- Cyclométhicone 5,0 g
- Alcool cétylique 4,0 g
- Stéarate de glycérol 3,0 g
- Glycérine 3,0 g
- Stéarate de polyéthylèneglycol oxyéthyléné à 40 moles d'oxyde d'éthylène, vendu sous la dénomination "MYRJ 52" par la Société ICI 2,0 g
- Myristate de myristyle 2,0 g
- Tristéarate de sorbitan 0,9 g
- Sel dipotassique de l'acide éthylène diamine tétracétique 0,09 g
- Composé de l'exemple 10 2,5 g

- Conservateur qs
- Eau qsp 100.0 g

Le composé de l'exemple 10 peut être remplacé par le composé de l'exemple 12.

EXEMPLE L

**EMULSION EAU-DANS-HUILE**

- Vaseline 14,25 g
- Huile de vaseline 12,20 g
- Lanoline hydrogénée 6,3 g
- Palmitate d'isopropyle 4,5 g
- Mélange d'octanoate de cétéaryl et de myristate d'isopropyle, commercialisé sous la dénomination "PCL LIQUIDE HUILE 2/066210" par la Société DRAGOCO 2.85 g
- Lanolate de magnésium 2,7 g
- Palmitate d'octoxyglycéryle 1,9 g
- Huile de palme 0,5 g
- 5-chloro-2-(2,4-dichlorophénoxy)phénol, commercialisé sous la dénomination "TRICLOSAN" par la Société CIBA-GEIGY 0.5 g
- Alcool de lanoline vendu sous la dénonciation "HARTOLAN" par la Société CRODA 0,43 g
- Composé de l'exemple 12 0.5 g
- Parfum qs
- Conservateur qs
- Eau qsp 100,0 g

Le composé de l'exemple 12 peut être remplacé par le composé de l'exemple 10.

**Revendications**

**1.** Composé de formule (I) :

$$CF_3 - (CF_2)_x - (CH_2)_y - \underset{(O)_w}{\overset{}{S}} - [C_5H_6N_2 R^+ X^-]_n - H \qquad (I)$$

dans laquelle :

w est égal à 0, 1 ou 2;
x est compris entre 2 et 10;
y est compris entre 0 et 5;
R désigne un radical éthyle, éthyle, hydroxyéthyle, benzyle;
$X^{\ominus}$ désigne un anion minéral ou organique;
n est un nombre entier ou décimal compris entre 1 et 15;

le groupement $[C_5H_6N_2R^+]$ représentant les structures suivantes prises en mélange ou séparément :

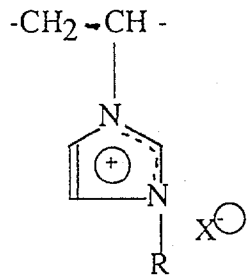

;

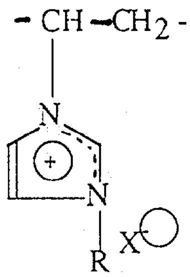

2. Composé selon la revendication 1, caractérisé par le fait que $X^{\ominus}$ désigne $Cl^-$, $Br^-$, $I^-$, $CH_3OSO_3^-$, $C_2H_5OSO_3^-$, $CH_3SO_3^-$,

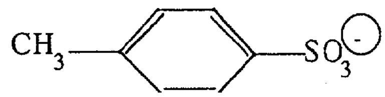

3. Composé selon la revendication 1, caractérisé par le fait que w est égal à O.

4. Procédé de préparation des composés de formule (I) selon l'une quelconque des revendications 1 à 3, caractérisé par le fait qu'il consiste à effectuer, sous atmosphère inerte, en milieu solvant inerte, une réaction d'addition radicalaire en présence d'un initiateur de radicaux libres, d'un mercaptan de formule :

$$CF_3 - (CF_2)_x - (CH_2)_y - SH$$

dans laquelle x et y ont la même signification indiquée dans la revendication 1, sur une ou plusieurs molécules de 1-vinylimidazole, pour obtenir un composé de formule (II) suivante :

$$CF_3 - (CF_2)_x - (CH_2)_y - S - (C_5H_6N_2)_n - H \qquad \text{(II)}$$

où $C_5H_6N_2$ signifie les structures suivantes prises en mélange ou séparément :

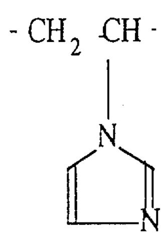

;

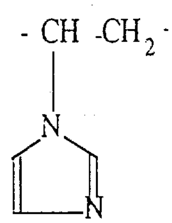

,

à quaterniser ensuite le composé ainsi obtenu en faisant réagir un alcoylant de formule RX, où R et X ont la même signification indiquée dans la revendication 1, en présence d'un solvant inerte puis, pour obtenir un composé de formule (I) où w est 1 ou 2, à oxyder le produit obtenu par de l'eau oxygénée à une température comprise entre 20 et 50°C.

5. Composé répondant à la formule (II) :

$$CF_3 - (CF_2)_x - (CH_2)_y - S - (C_5H_6N_2)_n - H \qquad \text{(II)}$$

dans laquelle :

x est compris entre 2 et 10;
y est compris entre 0 et 5;
n est un nombre entier ou décimal compris entre 1 et 15;
$C_5H_6N_2$ signifiant les structures suivantes prises en mélange ou séparémemt :

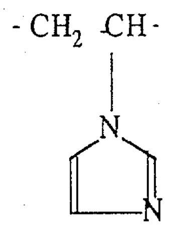
;
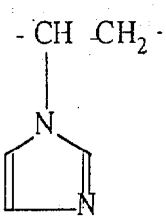

**6.** Utilisation des composés de formule (I) selon l'une quelconque des revendications 1 à 3, comme agents biocides ou agents conservateurs, dans des applications non thérapeutiques.

**7.** Composition pour le soin ou le traitement des matières kératiniques humaines, en particulier des cheveux, de la peau, des ongles ou des muqueuses, caractérisée par le fait qu'elle contient dans un milieu physiologiquement acceptable, au moins un composé de formule (I) selon l'une quelconque des revendications 1 à 3.

**8.** Composition selon la revendication 7, caractérisée par le fait qu'elle contient le composé de formule (I) dans des concentrations comprises entre 0,1 et 10% en poids par rapport au poids total de la composition.

**9.** Composition selon la revendication 7 ou 8, caractérisée par le fait qu'elle se présente sous forme de solutions aqueuses, hydroalcooliques ou alcooliques; d'émulsions se présentant sous forme de lait ou de crème; de mousse; de gel; de pâte; de stick; de spray; de dispersion vésiculaire ou conditionnée en aérosol.

**10.** Composition selon l'une quelconque des revendications 7 à 9, caractérisée par le fait qu'elle contient de l'eau, un mélange d'eau et de solvant(s) ou bien un solvant, le solvant étant physiologiquement acceptable et choisi parmi les monoalcools en $C_1$-$C_4$ ou les polyalcools; il est présent dans des proportions comprises entre 0 et 50%.

**11.** Composition selon l'une quelconque des revendications 7 à 10, caractérisée par le fait qu'elle contient en plus des huiles, des alcools gras, des silicones, des cires naturelles ou synthétiques, des tensio-actifs non-ioniques, cationiques, faiblement anioniques, amphotères ou zwittérioniques, des polymères d'origine naturelle ou synthétique, des conditionneurs, des stabilisants de mousse, des épaississants, des nacrants, des stérols, des sels, des filtres solaires, des conservateurs autres que ceux de formule (I), des parfums, des colorants, des hydratants.

**12.** Composition pharmaceutique destinée à être appliquée par voie topique pour traiter les maladies cutanées d'origine bactérienne, mycobactérienne ou dues à des implantations de levures pathogènes, caractérisée par le fait qu'elle est constituée d'une composition telle que définie dans l'une quelconque des revendications 7 à 11.

**13.** Procédé de traitement cosmétique des cheveux pour l'élimination des pellicules, caractérisé par le fait que l'on applique une composition selon l'une quelconque des revendications 7 à 11.

**14.** Composition de lavage et/ou de traitement des cheveux, avec rinçage et à séchage rapide, caractérisée par le fait qu'elle est constituée d'une composition selon l'une quelconque des revendications 7 à 11, contenant en plus des agents détergents et moussants et/ou des agents de traitement des cheveux usuels compatibles avec les composés de formule (I).

**15.** Utilisation des composés de formule (I) selon l'une quelconque des revendications 1 à 3, pour la préparation d'un médicament pour traiter les affections cutanées d'origine bactérienne, mycobactérienne ou dues à des implantations de levures pathogènes.

**16.** Utilisation des composés de formule (I) selon l'une quelconque des revendications 1 à 3, comme agent tensio-actif.

**17.** Utilisation des composés de formule (I) selon l'une quelconque des revendiçations 1 à 3, comme agent de conditionnement des cheveux ou de la peau.

**18.** Utilisation des composés de formule (I) selon l'une quelconque des revendications 1 à 3, comme agent de séchage rapide.

**19.** Utilisation des composés de formule (I) selon l'une quelconque des revendications 1 à 3, comme agent anti-regraissage de la peau ou des cheveux.

**Patentansprüche**

**1.** Verbindung der Formel (I) :

$$CF_3-(CF_2)_x-(CH_2)_y-\underset{(O)_w}{S}-[C_5H_6N_2R^+X^-]_n-H \qquad (I)$$

worin gilt:

w ist 0, 1 oder 2;
x beträgt 2 bis 10;
y beträgt 0 bis 5;
R bedeutet einen Methyl-, Ethyl-, Hydroxyethyl- oder Benzylrest;
$X^-$ bedeutet ein mineralisches oder organisches Anion;
n ist eine ganze Zahl oder Dezimalzahl von 1 bis 15;

die Gruppierung ($C_5H_6N_2R^+$) stellt die folgenden Strukturen, jeweils gemischt oder getrennt, dar:

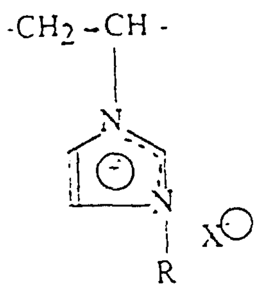 ; 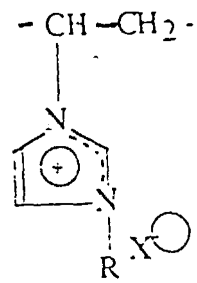

**2.** Verbindung gemäß Anspruch 1,
dadurch **gekennzeichnet**, daß
$X^-$ $Cl^-$, $Br^-$, $J^-$, $CH_3OSO_3^-$, $C_2H_5OSO_3^-$, $CH_3SO_3^-$
sowie

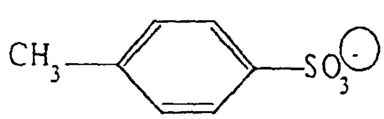

bedeutet.

**3.** Verbindung gemäß Anspruch 1,

dadurch **gekennzeichnet**, daß
w gleich 0 ist.

**4.** Verfahren zur Herstellung der Verbindungen der Formel (I) gemäß jedem der Ansprüche 1 bis 3, dadurch **gekennzeichnet**, daß man, unter Inertatmosphäre in inertem Lösungsmittelmilieu, in Gegenwart eines Initiators für freie Radikale eine radikalische Additionsreaktion eines Mercaptans der Formel:

$$CF_3 - (CF_2)_x - (CH_2)_y - SH$$

worin x und y die in Anspruch 1 angegebene Bedeutung haben, mit einem oder mehreren Molekülen von 1-Vinylimidazol durchführt, um eine Verbindung der folgenden Formel (II) zu erhalten:

$$CF_3 - (CF_2)_x - (CH_2)_y - S - (C_5H_6N_2)_n - H \qquad (II)$$

worin $C_5H_6N_2$ die folgenden Strukturen, jeweils in Mischung oder einzeln, bedeutet:

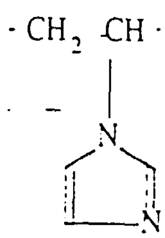
:
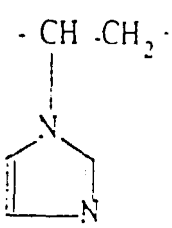

und daß man sodann die so erhaltene Verbindung quaterniert, indem man sie mit einem Alkylierungsmittel der Formel RX, worin R und X die in Anspruch 1 angegebene Bedeutung haben, in Gegenwart eines inerten Lösungsmittels reagieren läßt, worauf man, zum Erhalt einer Verbindung der Formel (I), worin w 1 oder 2 ist, das erhaltene Produkt mit einer sauerstoffhaltigen wässrigen Lösung bei einer Temperatur von 20 bis 50°C oxidiert.

**5.** Verbindung gemäß der Formel (II):

$$CF_3 - (CF_2)_x - (CH_2)_y - S - (C_5H_6N_2)_n - H \qquad (II)$$

worin gilt:

x beträgt 2 bis 10;
y beträgt 0 bis 5;
n ist eine ganze Zahl oder Dezimalzahl von 1 bis 15;
$C_5H_6N_2$ bedeutet die folgenden Strukturen, jeweils in Mischung oder einzeln:

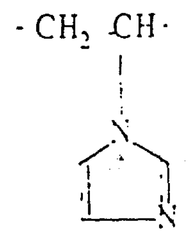

;

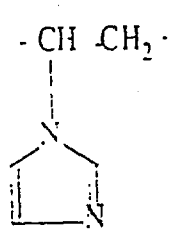

**6.** Verwendung der Verbindungen der Formel (I) gemäß einem der Ansprüche 1 bis 3 als biozide Mittel oder Konservierungsmittel in nicht-therapeutischen Anwendungsformen.

**7.** Zusammensetzung zur Pflege oder Behandlung menschlicher keratinischer Materien, insbesondere der Haare, der Haut, der Nägel oder von Schleimhäuten,
dadurch **gekennzeichnet**, daß
sie in einen physiologisch geeigneten Medium mindestens eine Verbindung der Formel (I) gemäß einem der Ansprüche 1 bis 3 enthält.

**8.** Zusammensetzung gemäß Anspruch 7,
dadurch **gekennzeichnet**, daß
sie die Verbindung der Formel (I) in Konzentrationen von 0,1 bis 10 Gew.% enthält, bezogen auf das Gesamtgewicht der Zusammensetzung.

**9.** Zusammensetzung gemäß Anspruch 7 oder 8,
dadurch **gekennzeichnet**, daß
sie in Form wässriger, hydroalkoholischer oder alkoholischer Lösungen, von Emulsionen in Form einer Milch oder Creme, eines Schaums, Gels, einer Paste, eines Stifts, Spray, einer bläschenartigen Dispersion oder zubereitet als Aerosol vorliegt.

**10.** Zusammensetzung gemäß einem der Ansprüche 7 bis 9,
dadurch **gekennzeichnet**, daß
sie Wasser, eine Mischung aus Wasser und Lösungsmittel(n) oder auch ein Lösungsmittel enthält, wobei das Lösungsmittel physiologisch geeignet und aus $C_{1-4}$-Monoalkoholen oder Polyalkoholen ausgewählt ist, wobei dieses in Mengenanteilen von 0 bis 50% vorhanden ist.

**11.** Zusammensetzung gemäß einem der Ansprüche 7 bis 10,
dadurch **gekennzeichnet**, daß
sie ausserdem Öle, Fettalkohole, Silicone, natürliche oder synthetische Wachse, nicht-ionische, kationische, schwach anionische, amphotere oder zwitterionische oberflächenaktive Mittel, Polymere natürlichen oder synthetischen Ursprungs, Konditioniermittel, Schaumstabilisiermittel,
Verdickungsmittel, Perlmuttglanzmittel, Sterole, Salze, Sonnenfilterstoffe, Konservierungsstoffe, die sich von denjenigen der Formel (I) unterscheiden, Parfüm-Produkte, Farbstoffe und Hydratisiermittel enthält.

**12.** Pharmazeutische Zusammensetzung zur Aufbringung auf topischem Wege zur Behandlung von Hauterkrankungen bakteriellen oder mykobakteriellen Ursprungs oder auf Grund von Implantationen aus pathogenen Verletzungen,
dadurch **gekennzeichnet**, daß
sie aus einer in jedem der Ansprüche 7 bis 11 definierten Zusammensetzung zusammengesetzt ist.

**13.** Verfahren zur kosmetischen Behandlung der Haare zur Beseitigung von Schuppen,
dadurch **gekennzeichnet**, daß
man eine Zusammensetzung gemäß jedem der Ansprüche 7 bis 11 aufbringt und anwendet.

**14.** Zusammensetzung zum Waschen und/oder Behandeln der Haare, unter Spülung und zur raschen Trocknung,
dadurch **gekennzeichnet**, daß
sie aus einer Zusammensetzung gemäß jedem der Ansprüche 7 bis 11 zusammengesetzt ist, welche ausserdem

Waschmittel und Schaummittel und/oder übliche, mit den Verbindungen der Formel (I) verträgliche Mittel zur Behandlung der Haare enthält.

**15.** Verwendung von Verbindungen der Formel (I) gemäß einem der Ansprüche 1 bis 3 zur Herstellung eines Medikaments zur Behandlung von Hauterkrankungen bakteriellen und mykobakteriellen Ursprungs oder in Folge von Implantationen aus pathogenen Verletzungen.

**16.** Verwendung von Verbindungen der Formel (I) gemäß einem der Ansprüche 1 bis 3 als oberflächenaktives Mittel.

**17.** Verwendung von Verbindungen der Formel (I) gemäß einem der Ansprüche 1 bis 3 als Konditioniermittel für Haare oder Haut.

**18.** Verwendung von Verbindungen der Formel (I) gemäß einem der Ansprüche 1 bis 3 als Mittel zur raschen Trocknung.

**19.** Verwendung von Verbindungen der Formel (I) gemäß einem der Ansprüche 1 bis 3 als Mittel gegen erneute Fettigkeit der Haut oder der Haare.

## Claims

**1.** Compound of formula (I):

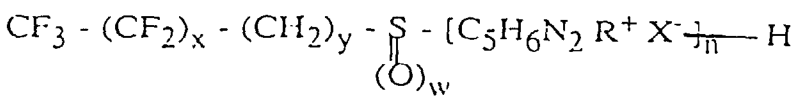

(I)

in which:

w is 0, 1 or 2;
x is between 2 and 10;
y is between 0 and 5;
R denotes a methyl, ethyl, hydroxyethyl or benzyl radical;
$X^{\ominus}$ denotes an inorganic or organic anion; and
n is an integer or decimal number between 1 and 15;

the $[C_5H_6N_2R^+]$ group representing the following structures, taken as a mixture or individually:

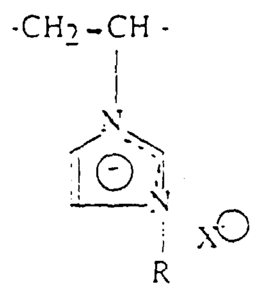

;

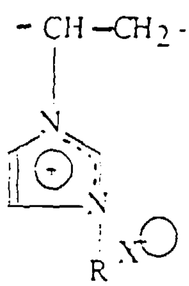

**2.** Compound according to Claim 1, characterized in that $X^{\ominus}$ denotes $Cl^-$, $Br^-$, $I^-$, $CH_3OSO_3^-$, $C_2H_5OSO_3^-$, $CH_3SO_3^-$,

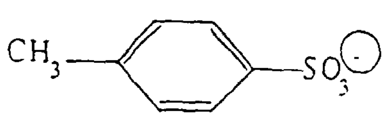

**3.** Compound according to Claim 1, characterized in that w is 0.

**4.** Process for the preparation of the compounds of formula (I) according to any one of Claims 1 to 3, characterized in that it consists in carrying out, under an inert atmosphere and in an inert solvent medium, a radical addition reaction, in the presence of a free radical initiator, of a mercaptan of formula:

$$CF_3 - (CF_2)_x - (CH_2)_y - SH$$

in which x and y have the same meaning indicated in Claim 1, with one or more molecules of 1-vinylimidazole, in order to obtain a compound of the following formula (II):

$$CF_3 - (CF_2)_x - (CH_2)_y - S - (C_5H_6N_2)_n - H \qquad (II)$$

where $C_5H_6N_2$ represents the following structures, taken as a mixture or individually:

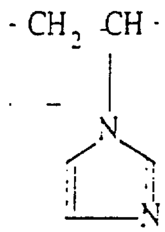
;
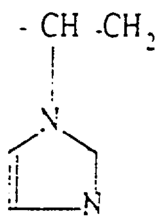
,

then in quaternizing the compound thus obtained by reacting with an alkylating agent of formula RX, where R and X have the same meaning indicated in Claim 1, in the presence of an inert solvent, and then, in order to obtain a compound of formula (I) where w is 1 or 2, in oxidizing the product obtained, using hydrogen peroxide at a temperature of between 20 and 50°C.

**5.** Compound corresponding to the formula (II) :

$$CF_3 - (CF_2)_x - (CH_2)_y - S - (C_5H_6N_2)_n - H \qquad (II)$$

in which

x is between 2 and 10;
y is between 0 and 5;
n is an integer or decimal number between 1 and 15;

$C_5H_6N_2$ representing the following structures, taken as a mixture or individually:

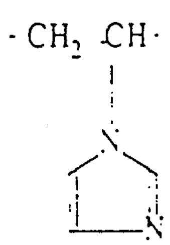
:
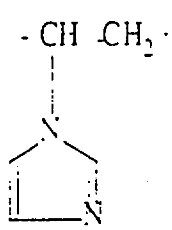

**6.** Use of the compounds of formula (I) according to any one of Claims 1 to 3 as biocidal agents or preservatives, in nontherapeutic applications.

**7.** Composition for the care or the treatment of human keratinous materials, in particular the hair, the skin, the nails or the mucosae, characterized in that it contains, in a physiologically acceptable medium, at least one compound of formula (I) according to any one of Claims 1 to 3.

**8.** Composition according to Claim 7, characterized in that it contains the compound of formula (I) in concentrations of between 0.1 and 10 % by weight, with respect to the total weight of the composition.

**9.** Composition according to Claim 7 or 8, characterized in that it is in the form of aqueous, aqueous-alcoholic or alcoholic solutions, emulsions in the form of a milk or cream, foam, gel, paste, stick, spray or vesicular dispersion or packaged as an aerosol.

**10.** Composition according to any one of Claims 7 to 9, characterized in that it contains water, a mixture of water and solvent(s) or a solvent, the solvent being physiologically acceptable and chosen from $C_1$-$C_4$ monoalcohols or polyalcohols; it is present in proportions of between 0 and 50 %.

**11.** Composition according to any one of Claims 7 to 10, characterized in that it also contains oils, fatty alcohols, silicones, natural or synthetic waxes, nonionic, cationic, weakly anionic, amphoteric or zwitterionic surfactants, polymers of natural or synthetic origin, conditioning agents, foam stabilizers, thickeners, agents for imparting a sheen, sterols, salts, sunscreens, preservatives other than those of formula (I), perfumes, colorants and moisturizers.

**12.** Pharmaceutical composition intended for topical application for the treatment of cutaneous diseases of bacterial or mycobacterial origin or due to implantations of pathogenic yeasts, characterized in that it consists of a composition as defined in any one of Claims 7 to 11.

**13.** Process for cosmetic treatment of hair for the elimination of dandruff, characterized in that a composition according to any one of Claims 7 to 11 is applied.

**14.** Composition for washing and/or treatment of hair, with rinsing and rapid drying, characterized in that it consists of a composition according to any one of Claims 7 to 11, additionally containing detergents and foaming agents and/or the conventional agents for treating hair, which are compatible with the compounds of formula (I).

**15.** Use of the compounds of formula (I) according to any one of Claims 1 to 3, for the preparation of a medicament for the treatment of cutaneous disorders of bacterial or mycobacterial origin or disorders due to implantations of pathogenic yeasts.

**16.** Use of the compounds of formula (I) according to any one of Claims 1 to 3, as surface-active agent.

**17.** Use of the compounds of formula (I) according to any one of Claims 1 to 3, as conditioning agent for the hair or the skin.

**18.** Use of the compounds of formula (I) according to any one of Claims 1 to 3, as agent for rapid drying.

**19.** Use of the compounds of formula (I) according to any one of Claims 1 to 3, as agent against regreasing of the skin or of the hair.